Europäisches Patentamt

**European Patent Office**

Office européen des brevets

(19)

(11) Veröffentlichungsnummer: **0 006 395**
**B1**

(12) **EUROPÄISCHE PATENTSCHRIFT**

(45) Veröffentlichungstag der Patentschrift:
**02.06.82**

(21) Anmeldenummer: **79810043.4**

(22) Anmeldetag: **25.05.79**

(51) Int. Cl.³: **A 01 K 11/00**, A 61 M 37/00

(54) **Verfahren und Einrichtung zum Kennzeichnen von Tieren.**

(30) Priorität: **08.06.78 CH 6240/78**

(43) Veröffentlichungstag der Anmeldung:
**09.01.80 Patentblatt 80/1**

(45) Bekanntmachung des Hinweises auf die Patenterteilung:
**02.06.82 Patentblatt 82/22**

(84) Benannte Vertragsstaaten:
**AT BE DE FR GB IT LU NL**

(56) Entgegenhaltungen:
**GB-A-1 443 815**
**GB-A-1 444 355**

(73) Patentinhaber: **Gübelin Ateliers AG, Grimselweg 5, CH-6005 Luzern (CH)**

(72) Erfinder: **Gasser, Rudolf, Bireggring 1, CH-6005 Luzern (CH)**

(74) Vertreter: **Bruderer, Werner, Schützenhausstrasse 5, CH-8330 Pfäffikon (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Verfahren und Einrichtung zum Kennzeichnen von Tieren

Die Erfindung betrifft ein Verfahren zum Anbringen von Kennzeichen an der Hautoberfläche von Tieren, bei dem vorgewählte Kennzeichen mittels Tätowiernadeln in die Haut eingestochen werden, wobei gleichzeitig mittels der Tätowiernadeln Tätowierflüssigkeit in die Einstiche eingebracht wird.

Die Erfindung betrifft auch eine Einrichtung zur Ausführung eines derartigen Verfahrens, welche mindestens eine durch einen Antrieb in eine schwingende Bewegung versetzbare Tätowiernadel aufweist, wobei die Nadelführung mit je einer Steuereinrichtung für die Impulsgebung und die Nadelbewegung versehen ist.

Apparate zum Tätowieren von Tieren sind in der Landwirtschaft bekannt. Sie dienen dazu, Schlacht- und Zuchttiere mit Identifikationszeichen zu kennzeichnen. Es ist eine Einrichtung in Form eines Handgerätes bekannt, welche mindestens zwei Tätowiernadeln aufweist, die gemeinsam in einem Tätowierkopf befestigt sind. Der Tätowierkopf kann in Richtung der Nadelachsen in Schwingung versetzt werden und wird von einem Gehäuse umgeben. Den Nadelspitzen wird automatisch Tätowierflüssigkeit zugeführt. Dies erfolgt durch Hohlräume in der Nadel oder durch Gleiten der Nadel durch eine dauernd getränkte Masse. Die gewünschten Zeichen werden den Tieren durch manuelles Bewegen des Gerätes in die Haut tätowiert. Nachteilig ist dabei, daß die Zeichen nicht einheitlich erscheinen und ein relativ großer Zeitaufwand notwendig ist. Eine entsprechende Einrichtung mit einer Mehrzahl von Tätowiernadeln ist aus der britischen Patentschrift Nr. 1 444 355 bekannt.

Es ist eine weitere Einrichtung bekannt (britische Patentschrift Nr. 1 443 815), mittels welcher Jungtiere, insbesondere Schweine, an den Ohren tätowiert werden. Dabei werden Tätowierstifte durch die Ohren gedrückt und nachfolgend automatisch Tätowierflüssigkeit in die Einstiche eingebracht. Die Tätowierstifte sind zu Stempeln zusammengefaßt, welche mit Steuerschiebern versehen sind. Das Ohr wird zwischen einem Tätowierkopf und einem Gegenhalter eingeklemmt, und die Tätowierflüssigkeit nach dem Rückzug der Nadeln aus dem Ohr mittels Vakuum aus einem Behälter in die Einstiche gesaugt oder durch Druck in die Einstiche gepreßt. Diese Art der Kennzeichnung hat den Nachteil, daß die Ohren im Verlaufe des Wachstums oft verletzt werden, wodurch die Kennzeichnung teilweise oder vollständig verloren geht. Andere Körperteile können auf diese Weise nicht gekennzeichnet werden.

Es ist Aufgabe der vorliegenden Erfindung, ein Verfahren und eine Einrichtung zum Kennzeichnen zu entwickeln, welche für das Tier möglichst schmerzlos sind, rasches Arbeiten ermöglichen, und eine zuverlässige und gleichartige Kennzeichnung der Tiere ermöglichen.

Die Erfindung, wie sie in den Ansprüchen gekennzeichnet ist, löst diese Aufgabe, indem das Anbringen der Kennzeichen in der Weise erfolgt, daß ein Teil eines Tätowiergerätes mit der Hauptoberfläche in Verbindung gebracht und zunächst zwischen Haut und Gerät ein Unterdruck erzeugt und die Haut an die Geräteoberfläche angesaugt wird. Darauf werden vorgewählte Kennzeichen mittels Tätowiernadeln in die Haut eingestochen, wobei gleichzeitig mittels der Tätowiernadeln Tätowierflüssigkeit in die Einstiche eingebracht wird. Nach Abschluß des Tätowiervorganges wird durch Einlassen von Luft zwischen Haut und Gerät der Unterdruck abgebaut und das Gerät von der Hautoberfläche, bzw. dem Tier, entfernt.

Während des Kennzeichnungsvorganges bestreichen die Nadeln das ihnen je durch Rahmen und Stege zugeordnete Kennzeichnungsfeld in Wechselbewegungen, welche von einer Seite des Feldes ausgehend etwa parallel zur Hauptoberfläche ausgeführt werden. An durch die Steuerung bestimmten Stellen führen die Nadeln Einstichbewegungen aus, und die Kennzeichen werden punktweise abgebildet. Die Steuerung kann sowohl mechanischer, elektromechanischer oder elektronischer Art sein und ist aus dem Stand der Technik bekannt. Die Zuführung der Tätowierflüssigkeit kann ebenfalls in bekannter Weise erfolgen.

Bei der Einrichtung zur Ausführung des Verfahrens ist am Gehäuse eines Tätowierapparates ein Aufsatz angeordnet, welcher mit mindestens einem Vakuumanschluß versehen ist. Im Aufsatz ist ein mit Saugöffnungen versehener Stützkörper angeordnet, wobei zwischen den Saugöffnungen die Nadelhalter mit den Tätowiernadeln angeordnet sind. In vorteilhafter Weise ist der Stützkörper mit einem vorstehenden, aus Rahmen und Stegen gebildeten Gitter versehen, wobei Rahmen und Stege nach außen offene, die Saugöffnungen bildende Nuten aufweisen. Rahmen und Stege bilden Durchgangsöffnungen, durch welche die Nadelhalter ragen und begrenzen das jeder Nadel zugeordnete Kennzeichnungsfeld.

Durch diese Gestaltung können eine oder beliebig viele Tätowiernadeln in einem Gerät zusammengefaßt werden. Die Grenze wird durch die Handhabbarkeit des gesamten Gerätes gesetzt. Die Ausgestaltung der Saugöffnungen kann ebenfalls in weiten Grenzen in Abhängigkeit von der Beschaffenheit der Haut und den gewünschten Haltekräften variiert werden. Die Ränder können flach, gerundet oder scharfkantig sein.

Die durch die Erfindung erreichten Vorteile sind im wesentlichen darin zu sehen, daß zwischen Gerät und Hautoberfläche während des Kennzeichnungsvorganges eine feste Verbindung hergestellt wird, ohne daß das Tier Schmerzempfindungen ausgesetzt wird. Zudem kann gleichzeitig eine Vielzahl von Zeichen angebracht werden, z. B. zwei Zahlengruppen von je fünf Zahlen. Durch den gewählten Bewegungsablauf der Nadeln ist auch während des Tätowiervorganges die Schmerzempfindung kaum vorhanden. Da die Haut eng und gespannt am Gerät anliegt, können die Stiche in der Tiefe genau bemessen und ihre Zahl auf das notwendige Minimum begrenzt werden, was ebenfalls in einer minimalen Schmerzempfindung resultiert.

Wird jeder Nadel ein Feld des Gebietes zugeordnet, ist ein Bewegen der Haut während des Kennzeichnens nicht möglich, und es werden sehr saubere und gleichmäßige Zeichen erzeugt. Die Einrichtung ermöglicht das Anbringen von Kennzeichen an Körperstellen, welche nicht durch Verletzung beschädigt werden. Dies sind z. B. bei Schweinen Schinken und Schulter.

Im folgenden wird die Erfindung anhand von lediglich eine Ausführungsform darstellenden Zeichnungen näher erläutert. Es zeigt

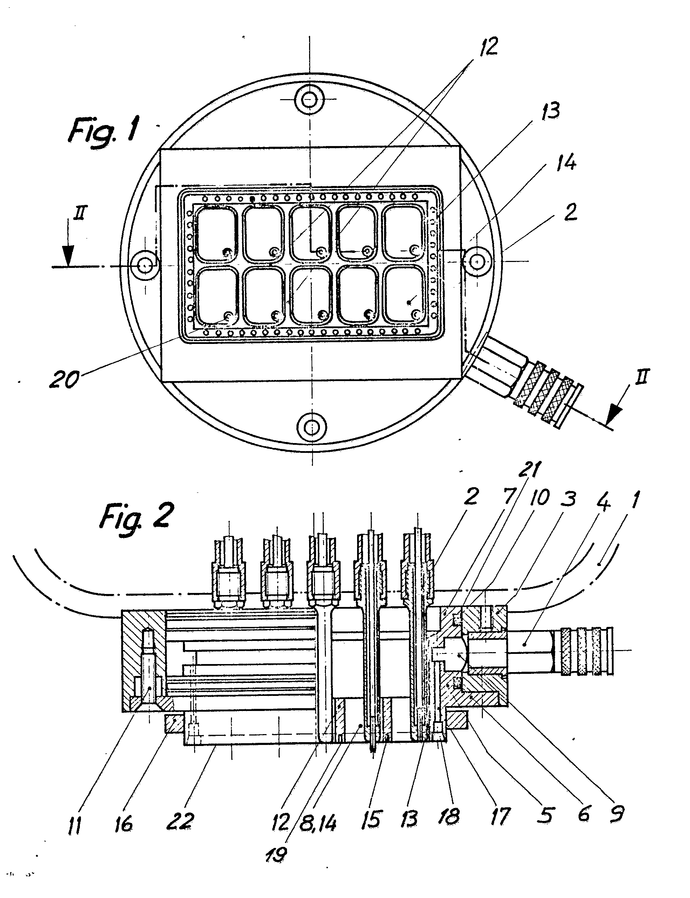

Fig. 1 eine Aufsicht auf einen erfindungsgemäßen Aufsatz an einem Tätowiergerät und Fig. 2 einen Schnitt nach Linie II-II in Fig. 1.

An einem in Fig. 2 strichpunktiert angedeuteten Tätowierapparat 1 ist ein Aufsatz 3 angebracht, welcher einen Stützträger 5, einen Vakuumanschluß 4 und Nadelhalter 2 mit Tätowiernadeln 20 umfaßt. Der Stützträger 5 ist mit einem vorstehenden Gitter 8 versehen, welches aus einem Rahmen 13 und Stegen 12 besteht. Der Stützträger 5 besteht aus einem Hohlzylinder 7 und einem Flansch 6, welcher mit Schrauben 11 mit dem Aufsatz 3 verbunden ist.

Der Hohlzylinder ist an seiner Außenseite mit einem als Vakuumkanal dienenden Ringkanal 9 versehen, welcher über den Vakuumanschluß 4 mit einer Vakuumleitung und einer Pumpe verbunden ist. Beidseitig des Ringkanals 9 sind Dichtungsrillen 21 zur Aufnahme von O-Ringen 10 vorgesehen.

Das Gitter 8 ist durch den Rahmen 13 und die Stege 12 so unterteilt, daß zwei Reihen von je fünf Durchgangsöffnungen 14 gebildet werden. Durch die Durchgangsöffnungen 14 ist je ein Nadelhalter 2 geführt, dessen vorderes Ende mit der Außenfläche des Gitters 8 bündig ist. Sowohl der Rahmen 13 als auch die Stege 12 sind mit Saugöffnungen in der Form von Nuten 15 und 18 versehen. Diese Nuten 15 und 18 umschließen die Kennzeichnungsfelder und sind über Bohrungen 17 mit dem Ringkanal 9 verbunden. Je nach Beschaffenheit der Haut und der Größe des angewendeten Unterdruckes werden die Randbereiche 19 der Saugöffnungen 15, 18 ausgestaltet. Im beschriebenen Beispiel sind sie flach ausgestaltet. Sie können aber auch scharfkantige, runde oder andere Formen aufweisen.

Zum Kennzeichnen wird die Haut des Tieres mit der Außenfläche 22 des Gitters 8 in Kontakt gebracht. Durch den erzeugten Unterdruck wird die Hautpartie fest an das Gitter 8 gesaugt und kann sich während des Kennzeichnungsvorganges nicht verschieben. Die Nadelhalter 2 bestreichen nun von einer Stelle ausgehend die Fläche der Durchgangsöffnung 14 in hin und her gehenden Bewegungen. An durch die Steuerungen bestimmten Stellen führen die Nadeln 20 Einstichbewegungen durch, welche über die Fläche 22 hinausgehen. Die Kennzeichnungsfelder werden von allen Nadelhaltern 2 in synchronen Bewegungen bestrichen. Sobald alle Zeichen abgebildet sind, wird zwischen Haut und Gitter 8 Luft eingelassen und die Saugwirkung abgebaut. Dies kann durch einseitiges Abheben des Gerätes 1 oder automatisch über Ventile erfolgen. Darauf können Gerät und Tierhaut wieder voneinander getrennt werden.

Der Tätowierapparat 1 umfaßt nicht gezeichnete allgemein bekannte Steuerungen und Antriebe für die Hin- und Her-Bewegungen der Nadelhalter 2 und die Schwingbewegungen der Tätowiernadeln 20.

In vorteilhafter Weise ist das Gitter 8 von einer Anschlagmarke 16 umgeben. Diese Marke kann durch nicht gezeichnete Verstellmittel in Bezug auf die Außenfläche 22 des Gitters 8 verstellt werden. Dadurch soll verhindert werden, daß die Randbereiche 19 der Saugöffnungen 15, 18 zu stark in die Haut eingepreßt werden.

Mit der beschriebenen beispielhaften Einrichtung können in ca. sechs Sekunden 1 bis 10 Zeichen gleichzeitig tätowiert werden. Durch die mittels Unterdruck zwischen Haut und Gitter 8 erzeugte feste Verbindung sind alle Zeichen sauber und genau. Die Einstichtiefe der Nadeln 20 kann in allen Feldern 14 genau eingehalten werden, da die Haut an der ganzen Außenfläche 22 des Gitters 8 anliegt. Als Resultat der kurzen Kennzeichnungszeit kann der Kennzeichnungsvorgang als praktisch schmerzfrei bezeichnet werden.

**Patentansprüche**

1. Verfahren zum Anbringen von Kennzeichen an der Hautoberfläche von Tieren, bei dem vorgewählte Kennzeichen mittels Tätowiernadeln in die Haut eingestochen werden, wobei gleichzeitig mittels der Tätowiernadeln Tätowierflüssigkeit in die Einstiche eingebracht wird, dadurch gekennzeichnet, daß ein Teil eines Tätowiergerätes mit der Hautoberfläche in Verbindung gebracht und zunächst zwischen Haut und Gerät ein Unterdruck erzeugt und die Haut an die Geräteoberfläche angesaugt wird, daraufhin vorgewählte Kennzeichen mittels Tätowiernadeln in die Haut eingestochen werden, wobei gleichzeitig mittels der Tätowiernadeln Tätowierflüssigkeit in die Einstiche eingebracht wird, und darauf durch Einlassen von Luft zwischen Haut und Gerät der Unterdruck abgebaut, und das Gerät von der Hautoberfläche entfernt wird.

2. Verfahren nach Patentanspruch 1, dadurch gekennzeichnet, daß die Nadeln während des Kennzeichnungsvorganges das ihnen jeweils zugeordnete Kennzeichnungsfeld in Wechselbe-

wegungen etwa parallel zur Hautoberfläche bestreichen und daß an durch die Steuerung bestimmten Stellen Einstichbewegungen ausgeführt und die Kennzeichen punktweise abgebildet werden.

3. Einrichtung zur Ausführung des Verfahrens nach Patentanspruch 1 oder 2, welche mindestens eine durch einen Antrieb in eine schwingende Bewegung versetzbare Tätowiernadel (20) aufweist, wobei die Nadelführung mit je einer Steuereinrichtung für die Impulsgebung und die Nadelbewegung in Wirkverbindung steht, dadurch gekennzeichnet, daß am Gehäuse (1) der Einrichtung ein Aufsatz (3) angeordnet ist, der mit mindestens einem Anschluß (4) für eine Vakuumleitung versehen ist, daß in dem Aufsatz ein Stützkörper (5) angeordnet ist, welcher mit dem Vakuumanschluß (4) verbundene Saugöffnungen (15, 18) aufweist, und daß zwischen Saugöffnungen (15, 18) die Nadelhalter (2) angeordnet sind.

4. Einrichtung nach Patentanspruch 3, dadurch gekennzeichnet, daß der Stützkörper (5) mit einem vorstehenden Gitter (8) versehen ist, welches einen Rahmen (13) und Stege (12) aufweist, wobei Rahmen (13) und Stege (12) mindestens teilweise mit den Saugöffnungen (15, 18) in Form von nach außen gerichteten Nuten versehen sind.

5. Einrichtung nach Patentanspruch 4, dadurch gekennzeichnet, daß durch die Stege (12) zwei Reihen von Durchgangsöffnungen (14) gebildet werden, durch welche die Nadelhalter (2) ragen.

6. Einrichtung nach einem der Patentansprüche 3 bis 5, dadurch gekennzeichnet, daß der Stützkörper (5) einen Ringkanal (9) aufweist und alle Saugöffnungen (15, 18) durch Bohrungen (17) mit dem Kanal (9) verbunden sind.

7. Einrichtung nach einem der Patentansprüche 3 bis 6, dadurch gekennzeichnet, daß um den Stützkörper (5) eine verstellbare Anschlagmarke (16) angeordnet ist.

## Claims

1. A method of applying identification marks to the surface of the hides of animals, wherein preselected identification marks are punctured in the hide by means of tattooing needles and wherein, at the same time, tattooing fluid is introduced into the punctures by means of the tattooing needles, characterized in that part of a tattooing implement is brought into contact with the surface of the hide and then reduced pressure is set up between the hide and the implement, and the hide is drawn by suction on to the surface of the implement, whereupon preselected identification marks are punctured in the hide by means of tattooing needles and, at the same time, tattooing fluid is introduced into the punctures by means of the tattooing needles, and thereafter the reduced pressure is cancelled out by the introduction of air between the hide and the implement, and the implement is removed from the surface of the hide.

2. A method according to claim 1, characterized in that during the marking process the needles are moved backwards and forwards approximately parallel with the surface of the hide and over the marking area associated with each of them, and in that puncturing movements are executed at places determined by the control means, and the identification marks are made in an punctiform manner.

3. Equipment for performing the method of claim 1 or claim 2, which comprises at least one tattooing needle (20) which can be set in oscillatory motion by a drive, each needle guide being in operative connection with a control device for releasing the impulse and moving the needle, characterized in that an attachment (3) ist arranged on the housing (1) of the equipment, which attachment is provided with at least one connector (4) for a vacuum lead, in that a support element (5), which has suction openings (15, 18) connected to the vacuum connector (4), is arranged in the attachment, and in that the needle holders (2) are arranged between the suction openings (15, 18).

4. Equipment according to claim 3, characterized in that the support element (5) is provided with a projecting grid (8), which comprises a frame (13) and webs (12), the frame (13) and webs (12) being provided, at least in part, with the suction openings (15, 18) in the form of outwardly directed grooves.

5. Equipment according to claim 4, characterized in that the webs (12) form two rows of passage openings (14) through which the needle holders (2) project.

6. Equipment according to any one of claims 3 to 5, characterized in that the support element (5) has an annular duct (9), and all of the suction openings (15, 18) are connected to the duct (9) by bores (17).

7. Equipment according to any one of claims 3 to 6, characterized in that an adjustable stop (16) is arranged around the support element (5).

## Revendications

1. Procédé d'application d'un marquage sur la surface de peau d'animaux, dans lequel des marques préalablement choisies sont piquées dans la peau au moyen d'aiguilles de tatouage, du liquide de tatouage étant simultanément introduit dans les piqûres au moyen des aiguilles de tatouage, caractérisé en ce qu'une partie d'un appareil de tatouage est amenée en liaison avec la surface de peau, et en ce que tout d'abord une pression réduite est produite entre la peau et l'appareil, et en ce que la peau est aspirée sur la surface de l'appareil, les marques préalablement choisies étant piquées dans la peau au moyen d'aiguilles de tatouage, du liquide de tatouage étant simultanément introduit dans les piqûres au moyen des aiguilles de tatouage, et en ce qu'ensuite la pression réduite est détruite par introduction d'air entre la peau et l'appareil et en ce que l'appareil est écarté de la surface de peau.

2. Procédé suivant la revendication 1, caractérisé en ce que les aiguilles balayent, pendent le processus de marquage, la zone de marquage qui leur est respectivement adjointe, suivant des mouvements alternés approximativement parallèlement à la surface de peau, et en ce que des mouvements de piqûres sont effectués aux endroits déterminés par la commande et en ce que le marquage est réalisé de manière punctiforme.

3. Dispositif pour la mise en oeuvre du procédé suivant l'une ou l'autre des revendications 1 et 2, qui présente au moins une aiguille de tatouage (20) déplaçable par un organe d'entraînement suivant un mouvement oscillatoire, le guidage d'aiguille étant en liaison active avec chaque fois un dispositif de commande pour la production d'impulsion et le mouvement de l'aiguille, caractérisé en ce que sur le boîtier (1) du dispositif est agencé un chapeau (3) qui est pourvu d'au moins un raccord (4) pour un conduit à vide, en ce que dans le chapeau est agencé un corps de support (5) qui présente des orifices d'aspiration (15, 18) reliés au raccord à vide (4), et en ce que les porte-aiguille (2) sont agencés entre les orifices d'aspiration (15, 18).

4. Dispositif suivant la revendication 3, caractérisé en ce que le corps de support (5) est pourvu d'une grille en saillie (8), qui présente un cadre (13) et des traverses (12), le cadre (13) et les traverses (12) étant pourvus au moins partiellement des orifices d'aspiration (15, 18), sous la forme de rainures orientées vers l'extérieur.

5. Dispositif suivant la revendication 4, caractérisé en ce que deux rangées d'ouvertures de passage (14), à travers lesquelles les porte-aiguille (2) passent, sont formées par les traverses (12).

6. Dispositif suivant l'une quelconque des revendications 3 à 5, caractérisé en ce que le corps de support (5) présente un canal annulaire (9) et en ce que lous les orifices d'aspiration (15, 18) sont reliés au canal (9) par des alésages (17).

7. Dispositif suivant l'une quelconque des revendications 3 à 6, caractérisé en ce qu'une marque d'arrêt ajustable (16) est agencée autour du corps de support (5).

Fig. 1

Fig. 2